(19) **Europäisches Patentamt / European Patent Office / Office européen des brevets**

(11) **EP 1 444 189 B1**

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(21) Anmeldenummer: **02787574.9**

(22) Anmeldetag: **05.11.2002**

(51) Int Cl.:
*C07C 47/04* (2006.01)     *C07C 47/058* (2006.01)
*C07C 45/82* (2006.01)     *C07C 45/79* (2006.01)

(86) Internationale Anmeldenummer:
**PCT/EP2002/012346**

(87) Internationale Veröffentlichungsnummer:
**WO 2003/040075 (15.05.2003 Gazette 2003/20)**

(54) **HOCHKONZENTRIERTE FORMALDEHYDLÖSUNG, DEREN HERSTELLUNG UND UMSETZUNG**

HIGHLY CONCENTRATED FORMALDEHYDE SOLUTION, PRODUCTION AND REACTION THEREOF

SOLUTION DE FORMALDEHYDE TRES CONCENTREE, PRODUCTION ET MISE EN REACTION DE LADITE SOLUTION

(84) Benannte Vertragsstaaten:
**AT BE BG CH CY CZ DE DK EE ES FI FR GB GR IE IT LI LU MC NL PT SE SK TR**

(30) Priorität: **05.11.2001 DE 10154187**
**21.08.2002 DE 10238248**

(43) Veröffentlichungstag der Anmeldung:
**11.08.2004 Patentblatt 2004/33**

(73) Patentinhaber: **BASF SE**
**67056 Ludwigshafen (DE)**

(72) Erfinder:
• **STRÖFER, Eckhard**
**68163 Mannheim (DE)**

• **SOHN, Martin**
**68229 Mannheim (DE)**
• **HASSE, Hans**
**70550 Stuttgart (DE)**
• **SCHILLING, Klemens**
**70550 Stuttgart (DE)**

(74) Vertreter: **Isenbruck, Günter**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**EP-A- 1 063 221     GB-A- 1 190 682**

**Beschreibung**

Die Erfindung betrifft wässrige Formaldehydlösungen enthaltend Formaldehyd in Form eines Gemisches aus monomerem Formaldehyd, Methylenglykol und Polyoxymethylenglykolen, ein Verfahren zu deren Herstellung, Verfahren zur Herstellung von Umsetzungsprodukten mit den erfindungsgemäßen wässrigen Formaldehydlösungen sowie die Verwendung der erfindungsgemäßen wässrigen Formaldehydlösungen. Formaldehyd ist eine wichtige Industriechemikalie und wird zur Herstellung zahlreicher Industrieprodukte und Verbrauchsartikel eingesetzt. In über 50 Industriezweigen wird derzeit Formaldehyd verwendet, im wesentlichen in Form von wässrigen Lösungen oder Formaldehyd enthaltenden Kunstharzen. Kommerziell erhältliche, wässrige Formaldehydlösungen weisen Gesamtkonzentrationen von 20 bis 55 Gew.-% Formaldehyd in Form von monomerem Formaldehyd, Methylenglykol und Polyoxymethylenglykolen auf. Somit wird in industriell angewendeten Synthesen, die unter Einsatz von wässrigen Formaldehydlösungen verlaufen, zusammen mit dem Formaldehyd eine große Menge Wasser eingetragen, die in der Synthese im allgemeinen nicht benötigt wird. Diese hohe Wasserlast bestimmt die Größe der Reaktoren, deren Peripherie sowie die Aufarbeitung der Produkte. Des weiteren muss das überschüssige Wasser als Abwasser behandelt und entsorgt werden. Gegebenenfalls ist es erforderlich, das Wasser unter erheblichem Energieeinsatz thermisch abzutrennen. Somit ist es wünschenswert, die Wasserlast in Synthesen, die den Einsatz wässriger Formaldehydlösungen erfordern, zu verringern, indem möglichst hochkonzentrierte wässrige Formaldehydlösungen eingesetzt werden.

Die Herstellung und Verwendung solcher hochkonzentrierter wässriger Formaldehydlösungen ist jedoch problematisch, da bei höher konzentrierten Lösungen, insbesondere bei niedrigen Temperaturen, Feststoffausfall auftritt. Wässrige Formaldehydlösungen mit mehr als 30 Gew.-% Formaldehyd werden bei einer Lagerung bei Raumtemperatur bereits trüb, da höhere Polyoxymethylenglykole ($HO(CH_2O)_nH$; $n \geq 8$) gebildet werden, die ausfallen. (Ullmann's Encyclopedia of Industrial Chemistry, Edition, 2000 electronic release, Formaldehyde; chapter 2 (physical properties), 2.2 (aqueous solutions), Seite 2, dritter Absatz). Bei höheren Temperaturen nimmt zwar die Löslichkeit der in der wässrigen Formaldehydlösung enthaltenen Produkte zu, jedoch erfolgt die unerwünschte Bildung von Ameisensäure durch Cannizzaro-Reaktion. Daher enthalten beispielsweise durch Destillation bei höheren Temperaturen und Drücken erzeugte hochkonzentrierte Formaldehydlösungen hohe Ameisensäuregehalte und sind somit durch niedrige pH-Werte gekennzeichnet.

Eine Verringerung der Wasserlast in Synthesen unter Einsatz von Formaldehyd kann zwar auch durch den Einsatz von Formaldehyd in fester Form als Paraformaldehyd oder Trioxan erreicht werden. Diese Produkte sind jedoch als Feststoffe verfahrenstechnisch wesentlich aufwendiger zu handhaben und in ihrer Herstellung erheblich teurer als wässrige Formaldehydlösungen.

GB 1,190,682 betrifft ein Verfahren zum Konzentrieren wässriger Formaldehydlösungen durch Destillation, wobei die Konzentration unter reduziertem Druck in mindestens einem Destillationsschritt durchgeführt wird, wobei wesentlich ist, dass die Destillationstemperatur in jedem Destillationsschritt unterhalb der Stabilitätstemperatur der konzentrierten Lösung liegt und die Destillation nur solange erfolgt, solange keine Trübung der Lösung auftritt. Im Anschluss an jeden Destillationsschritt wird die Lösung einer Alterungsphase ausgesetzt, die bei einer Temperatur oberhalb der Stabilitätstemperatur der konzentrierten Lösung liegt. Die Stabilitätstemperatur ist dabei die Temperatur, unterhalb der Polyoxymethylenglykole ausfallen. Mit diesem Verfahren können 75- bis 85 %ige wässrige Formaldehydlösungen hergestellt werden. Dieses Verfahren ist sehr aufwendig, da die hochkonzentrierten Lösungen gemäß der Beschreibung durch mehrstufige Konzentration und Alterung erhalten werden. Des weiteren enthält GB 1,190,682 keine Information, wie lange die hochkonzentrierten Lösungen stabil sind und ob die Zeitdauer ausreicht, um Folgereaktionen mit den konzentrierten Lösungen durchzuführen. Ferner bezieht sich GB 1,190,682 ausschliesslich auf Destillationsverfahren.

In dem Europäischen Patent EP-A 1 063 221 wird ein Verfahren zur Umsetzung einer Lösung, die ein Gemisch von mindestens zwei miteinander im chemischen Gleichgewicht stehenden chemischen Verbindungen enthält, mit einer Lösung wenigstens einer weiteren chemischen Verbindungen beschrieben, wobei die Lösung der im chemischen Gleichgewicht befindlichen Stoffe in zwei Fraktionen mit Nichtgleichgewichtszusammensetzung aufgetrennt und vor der vollständigen Wiedereinstellung des chemischen Gleichgewichts mit der anderen chemischen Verbindung umgesetzt wird. Genaue Angaben über die Kettenlänge der Polyoxymethylenglykole und ihren Anteil an der Gesamtzusammensetzung sowie deren Einfluß auf den Zeitraum, über den diese Lösungen stabil sind, werden nicht gemacht.

Aufgabe der vorliegenden Erfindung ist es daher, hochkonzentrierte Formaldehydlösungen bereitzustellen, die leicht handhabbar und einfach in einem ein- oder mehrstufigen Verfahren herstellbar sind, und die in Synthesen, die unter Einsatz wässriger Formaldehydlösungen erfolgen, eingesetzt werden können, so dass die Wasserlast in diesen Synthesen verringert wird.

Die Lösung dieser Aufgabe geht aus von wässrigen Formaldehydlösungen enthaltend Formaldehyd in Form von monomerem Formaldehyd, Methylenglykol und Polyoxymethylenglykolen in einer Gesamtkonzentration x von $\geq 65$ Gew.-%.

Die erfindungsgemäßen hochkonzentrierten wässrigen Formaldehydlösungen sind dann dadurch gekennzeichnet, dass die mittlere Molmasse $\overline{M}$ der Polyoxymerhylenglykole in Abhängigkeit von der Formaldehydkonzentration gleich oder kleiner den gemäß Formel I erhaltenen Werten ist:

$$\left(\frac{\overline{M}}{g/mol}\right) = 48 + 6{,}589 \cdot 10^{-1} \cdot \left(\frac{x}{Gew.-\%}\right) + 4{,}725 \cdot 10^{-2} \cdot \left(\frac{x}{Gew.-\%}\right)^{2} - 3{,}434 \cdot 10^{-3} \cdot \left(\frac{x}{Gew.-\%}\right)^{3}$$

$$+ 9{,}625 \cdot 10^{-5} \cdot \left(\frac{x}{Gew.-\%}\right)^{4} - 1{,}172 \cdot 10^{-6} \cdot \left(\frac{x}{Gew.-\%}\right)^{5} + 5{,}357 \cdot 10^{-9} \cdot \left(\frac{x}{Gew.-\%}\right)^{6}$$

$$(I)$$

darin bedeuten:

$\overline{M}$   mittlere Molmasse

x   Gesamtkonzentration an Formaldehyd in Form von monomerem Formaldehyd, Methylenglycol und Polyoxymethylenglycolen in Gew.-% (Formaldehydgesamtkonzentration)

1   Die Formel I ist im Bereich einer Gesamtkonzentration x von 0 bis 95 Gew.-% gültig.

Die mittlere Molmasse $\overline{M}$ der Reaktionsprodukte des Formaldehyds mit Wasser (Methylenglykol und Polyoxymethylenglykole) in Abhängigkeit von der Formaldehydkonzentration ist gleich oder kleiner der in der folgenden Tabelle angegebenen Werte, die im Folgenden für ausgewählte Formaldehydkonzentrationen angegeben sind:

| x/Gew.-%[1] | $\overline{M}$ |
|---|---|
| 65 | 109,9 |
| 75 | 132,4 |
| 85 | 181,2 |

1) Formaldehydgesamtkonzentration

Die Werte bei anderen Temperaturen und Formaldehydkonzentrationen ergeben sich durch Anwendung von Formel I. Bevorzugt werden Mischungen, bei denen die Werte für die mittlere Molmasse $\overline{M}$ der Reaktionsprodukte des Formaldehyds mit Wasser die Werte gemäß Formel I um mindestens 5% unterschreiten. Besonders bevorzugt sind Mischungen bei denen die Werte gemäß Formel I um mindestens 10% unterschritten werden, und ganz besonders bevorzugt sind Mischungen bei denen die Werte um mindestens 20% unterschritten werden. Die erfindungsgemäßen Lösungen können gegebenenfalls noch andere Inhaltsstoffe wie Stabilisatoren enthalten.

Im Gegensatz dazu liegt in einer kommerziell erhältlichen Formaldehydlösung die mittlere Molmasse $\overline{M}$ der Polyoxymethylenglykole in Abhängigkeit von Temperatur und Formaldehydkonzentration bei den in der folgenden Tabelle angegebenen Werten, die im Folgenden für ausgewählte Konzentrationen angegeben sind:

Kommerziell erhältliche Formaldehydlösungen

| x / Gew.-%[1] | $\overline{M}$ |
|---|---|
| 37 | 76,8 |
| 50 | 88,8 |

1) Formaldehydgesamtkonzentration

| $X_{Formaldehyd}$ / Gew.-% [1] | $X_{Methanol}$ / Gew.-% [2] | $\overline{M}$ |
|---|---|---|
| 37 | 1,5 | 77 |
| 49 | 2 | 83 |

1) Formaldehydgesamtkonzentration

2) Methanolgesamtkonzentration

Die kommerziell erhältlichen Lösungen gemäß der obigen Tabelle können gegebenenfalls noch andere Inhaltsstoffe wie Stabilisatoren in geringen Mengen enthalten.

Die mittlere Konzentration des Methylenglykols und der Polyoxymethylenglykole kann z.B. mit der $^1$H- oder $^{13}$C-NMR-Spektroskopie mit in der Literatur beschrieben Methoden [Hahnenstein, I., Albert, M., Hasse, H., Kreiter, C.G., Maurer, G., NMR Spectroscopic and Densimetric Study of Reaction Kinetics of Formaldehyde Polymer Formation in Water, Deuterium Oxide, and Methanol, Ind. Eng. Chem. Res. (1995) 34, 440-450] ermittelt werden. Die Formaldehydgesamtkonzentration einer wässrigen Formaldehydlösung kann mit gängigen und in der Literatur beschriebenen Methoden wie z.B. der Sulfit-Titration [J. F. Walker, Formaldehyde, 2nd edition, Reinhold Publ. Comp., New York, 1953, S. 382 ff.] bestimmt werden.

Die erfindungsgemäßen Formaldehydlösungen sind einfach, ohne großen Aufwand in kurzer Zeit herstellbar. Es ist keine Alterung der Lösungen während der Herstellung erforderlich, bzw. eine Alterung ist unerwünscht. Des weiteren ist die Herstellung der erfindungsgemäßen Formaldehydlösungen sowohl ausgehend von Gleichgewichtslösungen als auch ausgehend von Nichtgleichgewichtslösungen möglich.

Durch den Einsatz dieser erfindungsgemäßen hochkonzentrierten wässrigen Formaldehydlösungen kann

a) der Anteil inerter Komponenten (Wasserlast) vermindert und somit die Raum-ZeitAusbeute (RZA) erhöht sowie die Investitionskosten durch die Verwendung kleinerer Vorrichtungen gesenkt werden,

b) die Menge des anfallenden Abwassers reduziert werden und

c) eine Energieeinsparung bei der gegebenenfalls nötigen thermischen Abtrennung des Wassers erzielt werden.

So werden beispielsweise in der Produktion von 20,8 t/h Methandiphenyldiamin (MDA) zur Produktion von 26,2 t/h Methylendiphenyldiisocyanat (MDI), entsprechend einer 200 kt/a-MDI-Anlage, 8,2 t/h 50 Gew.-%-ige wässrige Formaldehydlösung benötigt. Dies entspricht einer Wasserlast von 4,1 t/h. Durch den Einsatz einer 65 Gew.-%-igen wässrigen Formaldehydlösung kann diese Wasserlast fast halbiert werden auf 2,2 t/h.

Gemäß der vorliegenden Erfindung wurde gefunden, dass die Bereitstellung von Formaldehydlösungen mit Formaldehyd in Form von monomerem Formaldehyd, Methylenglykol und Polyoxymethylenglykolen in einer Gesamtkonzentration von $\geq$ 65 Gew.-% bei Temperaturen von im allgemeinen -5 bis 150 °C, bevorzugt von 10 bis 100 °C, besonders bevorzugt bei Raumtemperatur bis 50°C möglich ist, ohne dass Feststoffausfall (Feststofffreiheit) auftritt. Dabei ist unter Feststofffreiheit im Sinne der vorliegenden Erfindung ein Feststoffgehalt in den erfindungsgemäßen wässrigen Formaldehydlösungen von im allgemeinen < 1 Gew.-%, bevorzugt < 0,5 Gew.-%, besonders bevorzugt < 0,1 Gew.-% zu verstehen.

Es wurde gefunden, dass der Feststoffausfall in hochkonzentrierten wässrigen Formaldehydlösungen dadurch vermieden werden kann, dass nicht - wie im Stand der Technik bisher angenommen - die Menge der Polyoxymethylenglykole verringert wird, sondern deren mittlere Kettenlänge (die mit der mittleren Molmasse korreliert). Auf diese Weise ist es möglich, hochkonzentrierte wässrige Formaldehydlösungen bereitzustellen, die lange genug stabil sind (kein Feststoffausfall), um eine Umsetzung mit geeigneten Verbindungen - auch bei Temperaturen, bei denen in solchen hochkonzentrierten Formaldehydlösungen üblicherweise Feststoffausfall auftritt - durchzuführen.

Unter einer wässrigen Formaldehydlösung ist gemäß der vorliegenden Erfindung eine Formaldehydlösung zu verstehen, die mindestens 5 Gew.-%, bevorzugt mindestens 10 Gew.-% Wasser enthält.

Diese erfindungsgemäßen wässrigen Formaldehydlösungen zeichnen sich bevorzugt dadurch aus, dass bei Temperaturen von im allgemeinen -5 bis 180°C, bevorzugt 10 bis 100 °C, besonders bevorzugt von Raumtemperatur bis 50°C - also bei Temperaturen, bei denen üblicherweise die Reaktionen mit Formaldehyd durchgeführt werden - über einen Zeitraum von mindestens 1 Minute, bevorzugt mindestens 5 Minuten, besonders bevorzugt mindestens 1 Stunde kein Feststoffausfall auftritt.

Somit sind die erfindungsgemäßen wässrigen Formaldehydlösungen überall dort einsetzbar, wo Reaktionen mit geeigneten Verbindungen in dem genannten Zeitraum ablaufen.

Ein weiterer Vorteil der erfindungsgemäßen Lösungen ist, dass sie auch bei hohen Formaldehydgesamtkonzentrationen geringe Ameisensäurekonzentrationen aufweisen können. Der pH-Wert einer kommerziell erhältlichen 49 gew.-%igen Formaldehydlösung (mit 1,0 bis 2,0 Gew.-% Methanol als Stabilisator) ist üblicherweise 3,0 bis 3,5 bei 50°C. Erfindungsgemäße Lösungen mit deutlich höheren Formaldehydgehalten, z.B. 65 Gew.-%, können wesentlich höhere pH-Werte aufweisen (z.B. 4,0 bis 6,0) und damit weniger Ameisensäure enthalten.

Die Verwendung von Stabilisatoren zur Unterdrückung des Feststoffausfalls, die bei chemischen Reaktionen gegebenenfalls stören können, ist in den erfindungsgemäßen wässrigen Formaldehydlösungen nicht erforderlich. Es ist jedoch möglich, Stabilisatoren einzusetzen. Dabei können beliebige Stabilisatoren eingesetzt werden. Ist der Einsatz von Stabilisatoren erwünscht, so werden vorzugsweise Stabilisatoren ausgewählt aus Alkoholen, insbesondere Methanol, Ethanol, Propanol, Butanol; sowie Harnstoff und Melamin eingesetzt.

Da Formaldehyd hoch reaktiv ist, ist dieser Zeitraum ausreichend, um die erfindungsgemäßen wässrigen Formaldehydlösungen in Synthesen, bevorzugt in industriell anwendbaren Synthesen, einzusetzen.

Die vorliegende Erfindung ermöglicht somit die Bereitstellung von hochkonzentrierten wässrigen Formaldehydlösungen, die Formaldehyd in Form von monomerem Formaldehyd, Methylenglykol und Polyoxymethylenglykolen in einer Gesamtkonzentration von $\geq$ 65 Gew.-%, bevorzugt von $\geq$ 70 Gew.-%, besonders bevorzugt von $\geq$ 75 Gew.-% aufweisen.

Die Herstellung der erfindungsgemäßen wässrigen Formaldehydlösungen erfolgt durch Entzug von Wasser oder einer Wasser enthaltenden Mischung, bevorzugt durch schnellen Entzug im allgemeinen in 1 Sekunde bis 5 Stunden, bevorzugt 5 Sekunden bis 1 Stunde, besonders bevorzugt 10 Sekunden bis 30 Minuten Die erhaltenen erfindungsgemäßen wässrigen Formaldehydlösungen können überraschenderweise und entgegen des allgemeinen Stands des Wissens über einen für eine chemische Umsetzung hinreichend langen Zeitraum von im allgemeinen mindestens 1 Minute, bevorzugt mindestens 5 Minuten, besonders bevorzugt mindestens 1 Stunde aufrechterhalten werden, ohne das es zu einem Feststoffausfall kommt. Insbesondere ist keine Alterung bei erhöhten Temperaturen nötig. Die Erhöhung der Temperatur ist im allgemeinen sogar unerwünscht.

Die Herstellung der erfindungsgemäßen wässrigen Formaldehydlösungen erfolgt durch folgendes Verfahren:

einstüfige thermische Auftrennung einer wässrigen Formaldehydlösung enthaltend Wasser und 5 bis 65 Gew.-% eines Ausgangsgemisches von Formaldehyd in Form von monomerem Formaldehyd, Methylenglykol und Polyoxymethylenglykolen sowie gegebenfalls weitere Komponenten wie Stabilisatoren, wobei die wässrige Formaldehydlösung zumindest teilweise verdampft wird, in wenigstens zwei Fraktionen, in denen unterschiedliche Verbindungen des Gemisches im Vergleich zu dem Ausgangsgemisch im Gemisch angereichert sind,

wobei in wenigstens einer der wenigstens zwei Fraktionen Wasser im Vergleich zum Ausgangsgemisch abgereichert ist, so dass der Formaldehyd in Form von monomerem Formaldehyd, Methylenglykol und Polyoxymethylenglykolen in einer Gesamtkonzentration von $\geq$ 65 Gew,-%, bevorzugt $\geq$ 70 Gew.-%, besonders bevorzugt $\geq$ 75 Gew.-% vorliegt, wobei im Anschluß an die Auftrennung keine Alterung der Fraktion, worin Formaldehyd in Form von monomeren Formaldehyd, Methylenglykol und Polyoxymethylenglykolen in einer Gesamtkonzentration von $\geq$ 65 Gew.-% vorliegt, bei erhöhten Temperaturen erfolgt.

Die Komponenten der eingesetzten wässrigen Formaldehydlösungen können sich bei dem erfindungsgemäßen Verfahren im Gleichgewichts- oder im Nichtgleichgewichtszustand befinden.

Bei den wenigstens zwei Fraktionen handelt es sich bevorzugt um eine erste im allgemeinen leichtsiedende Fraktion, enthaltend monomeren Formaldehyd und Methylenglykol und eine zweite im allgemeinen höher siedende Fraktion, enthaltend Wasser, monomeren Formaldehyd, Methylenglykol und Polyoxymethylenglykole. Die erste Fraktion enthält des weiteren das aus der wässrigen Formaldehydlösung entfernte Wasser.

Die erfindungsgemäßen wässrigen Formaldehydlösungen werden mit Hilfe geeigneter Trennverfahren gewonnen.

Die Auftrennung erfolgt durch ein Trennverfahren, das mindestens einen Schritt enthält, bei dem die wässrige Formaldehydlösung zumindest teilweise verdampft wird; es erfolgt eine thermische Auftrennung. Dabei erfolgt die thermische Auftrennung einstufig im Gleich- oder im Gegenstrom. Dies kann z.B. in einer Destillationseinrichtung geschehen.

In einer besonders bevorzugten Ausführungsform des erfindungsgemäßen Verfahrens erfolgt die Abtrennung von Wasser oder einer Wasser enthaltenden Mischung schnell. Dabei erfolgt die Abtrennung in einem Zeitraum von 1 Sekunde bis 5 Stunden, bevorzugt von 5 Sekunden bis 1 Stunde, besonders bevorzugt von 10 Sekunden bis 30 Minuten.

Besonders bevorzugt wird die zumindest teilweise Verdampfung in Form einer einstufigen Verdampfung durchgeführt. Als Verdampferbauarten sind beispielsweise Selbstumlauf-, Zwangsumlauf-, Kletterfilm-, Dünnschicht- und Fallfilmverdampfer sowie Rührkessel geeignet. Bevorzugt wird in dem erfindungsgemäßen Verfahren ein Filmverdampfer eingesetzt. Ein geeigneter Filmverdampfer ist z.B. in EP-A 1 063 221 offenbart. Dabei handelt es sich um einen Dünnschichtverdampfer. Des weiteren wird in dem erfindungsgemäßen Verfahren bevorzugt ein Fallfilmverdampfer eingesetzt.

In einer weiteren bevorzugten Ausführungsform wird die zumindest teilweise Verdampfung in einem Wendelrohr- bzw. Schlangenrohrverdampfer durchgeführt. Geeignete Wendelrohr- bzw. Schlangenrohrverdampfer sind im Chem. Ing. Tech. 68 (6), 1996, Seiten 706 bis 710 und in Chem. Ing. Tech. 42 (6), 1970, Seiten 349 bis 354 offenbart. Bei Einsatz eines Wendelrohr- bzw. Schlangenrohrverdampfers wird die Ausgangslösung unter Druck zu einem Vorwärmer geführt, dort aufgeheizt und anschließend unter Dampfbildung entspannt. Im folgenden beheizten Wendelrohr wird die Lösung dann bis zum Endprodukt aufkonzentriert.

Besonders bevorzugt wird in dem erfindungsgemäßen Verfahren ein Filmverdampfer ausgewählt aus Dünnschichtverdampfer, Wendelrohr- bzw. Schlangenrohrverdampfer und Fallfilmverdampfer eingesetzt.

Weiterhin ist es möglich, die zumindest teilweise Verdampfung in einer Kolonne, bevorzugt in einer Reaktionskolonne, durchzuführen. Beispielsweise kann eine kommerziell erhältliche Formaldehydlösung bei einer Temperatur von im allgemeinen -5 bis 150 °C, bevorzugt von 10 bis 100 °C, ganz besonders bevorzugt von Raumtemperatur bis 50°C in einer Reaktionskolonne entspannt werden, in dem man bevorzugt den Druck, unter dem die Lösung steht, stark vermindert, wodurch die höheren Homologen des Formaldehyds in der Lösung verbleiben und monomeres Formaldehyd, Wasser und gegebenenfalls Methylenglykol verdampft. Dabei ist die Temperatur abhängig von dem Druck. Dieser beträgt im allgemeinen 1 mbar bis 40 bar, bevorzugt 10 mbar bis 11 bar, besonders bevorzugt 50 mbar bis 1 bar.

In einer Ausführungsvariante des erfindungsgemäßen Verfahrens erfolgt das schnelle Abdampfen der Leichtsieder in einem Filmverdampfer.

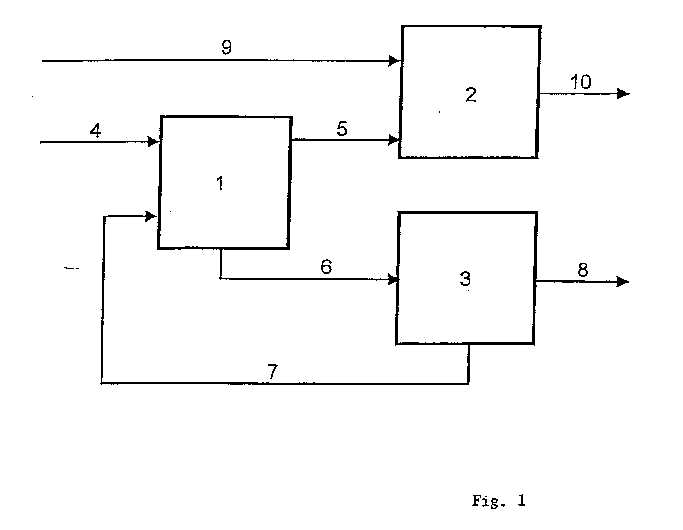

In Figur 1 ist schematisch der Ablauf eines erfindungsgemäßen Verfahrens dargestellt. In Figur 1 bedeuten:

1    Filmverdampfer
2    Hauptreaktorsystem
3    Trenneinrichtung
4    Rohlösung
5    erwünschte Fraktion (erfindungsgemäße wässrige Formaldehydlösung)
6    Restfraktion (Leichtsiederfraktion)
7    zurückgeführte Lösung
8    Ausschleusestrom
9    Edukte
10    Produkte

Dabei sind die Apparate/Ströme 3, 7, 8 nur optional.

Über eine Zuleitung wird eine Formaldehyd enthaltene wässrige Rohlösung 4, z.B. handelsübliche 20 bis 55 Gew.-%-ige wässrige Formaldehydlösung, zugeführt. Diese Lösung enthält mehrere, miteinander im allgemeinen aber nicht notwendigerweise im chemischen Gleichgewicht stehende Komponenten: Wasser, monomeres Formaldehyd (HCHO); Methylenglykol ($CH_2(OH)_2$), welches aus Formaldehyd durch Reaktion mit Wasser entsteht, sowie Polyoxymethylenglykole ($HO(CH_2O)_nH$; mit n = 2 bis 30), welche durch Kondensation des Methylenglykols entstehen.

Diese Rohlösung 4 wird einem Filmverdampfer 1 zugeführt. In diesem wird sie, bevorzugt durch schnellen Wasserentzug, in eine erwünschte Fraktion 5 (erfindungsgemäße wässrige Formaldehydlösung) und eine Restfraktion 6 (Leichtsiederfraktion, enthaltend monomeren Formaldehyd, gegebenenfalls Methylenglykol und Wasser) aufgetrennt. Diese erwünschte Fraktion (erfindungsgemäße wässrige Formaldehydlösung) wird anschließend im allgemeinen einem Hauptreaktorsystem 2 zugeführt, wo sie mit weiteren Edukten 9 zu Produkten 10 umgesetzt wird.

Die Restfraktion 6 kann in vielen Fällen an anderer Stelle in Gesamtprozessen weiter verwendet werden. In diesem Fall entfallen die Apparate/Ströme 3, 7, 8. Insbesondere eignet sich die Restfraktion 6 sich zur Einspeisung in den Absorber bei üblichen Verfahren zur Herstellung von Formaldehyd aus Methanol und ersetzt dort vorteilhaft Waschwasser. Soll die Restfraktion 6 nicht wie oben beschrieben anderweitig verwendet werden, so bietet sich das in der Figur 1 gezeigte Verfahren an. In diesem wird die Fraktion 6 einer Trenneinrichtung 3 zugeführt, in der mit geeigneten Mitteln (z.B. durch Destillation oder Extraktion) Wasser entzogen wird (Wasserausschleusung 8). Die so entstandene Lösung 7 wird danach der zugeführten Rohlösung 4 beigemischt und erneut in den Filmverdampfer 1 eingeleitet. Während bei der Trennung 1 niedrige Verweilzeiten vorteilhaft sind, sind die Verweilzeiten bei der Trennung 3 unkritisch. Im allgemeinen werden Verweilzeiten, wie sie bei technischen Destillationen auftreten (im Bereich von einigen Minuten bis einigen Stunden), geeignet sein. Bevorzugt werden sogar hohe Verweilzeiten. Es ist insbesondere möglich, aber keineswegs notwendig, zwischen die Apparate 1 und 3 bzw. 3 und 1 (Ströme 6 bzw. 7) Verweilzeitbehälter zwischenzuschalten.

Geeignete Betriebsbedingungen für eine thermische Auftrennung in Apparat 1 sind eine Temperatur zwischen im allgemeinen 5°C und 150°C, bevorzugt zwischen 10°C und 100°C, im allgemeinen bei einem Absolutdruck zwischen 0,1 mbar und 40 bar. Bei Einsatz eines Filmverdampfers sind Temperaturen zwischen 20°C und 100°C bei Normaldruck besonders bevorzugt. Neben dem in EP-A 1 063 221 offenbarten Dünnfilmverdampfer ist es auch möglich, einen Filmverdampfer ohne mechanische Beeinflussung des Flüssigkeitsfilms auf der Verdampfungsfläche einzusetzen. Die Wärmeübertragungsfläche solcher Fallfilm- oder Fallstromverdampfer kann dabei als Rohre oder Platten ausgebildet sein. Verschiedene Betriebsweisen eines Film verdampfers sind in EP-A 1 063 221 sowie in Chem. Ing. Tech. 42 (6), 1970, Seiten 349 bis 354 und Chem. Ing. Tech. 68 (6), 1996, Seiten 706 bis 710 aufgeführt.

Bei einer Auftrennung in einer Destillationseinrichtung durch ein stufige Verdampfung, z.B. durch Flash sind Temperaturen von im allgemeinen 50 bis 180 °C, bevorzugt > 100 bis 180 °C geeignet, im allgemeinen bei einem Druck von 0,2 bar bis 10 bar, da so eine bevorzugte schnelle Abtrennung des Wassers erreicht wird.

Für die erfindungsgemäßen wässrigen Formaldehydlösungen sind eine Vielzahl von Anwendungsmöglichkeiten gegeben. Grundsätzlich können die erfindungsgemäßen wässrigen Formaldehydlösungen in jedem Verfahren eingesetzt werden, in dem eine wässrige Formaldehydlösung benötigt wird. Dabei sind keine wesentlichen Verfabrensänderungen erforderlich, sondern es kann ein einfacher Austausch der bisher eingesetzten Formaldehydlösungen erfolgen, um den Vorteil der verminderten Wasserlast zu nutzen.

Ein weiterer Gegenstand der vorliegenden Anmeldung ist daher ein Verfahren zur Herstellung von monomeren, oligomeren und polymeren Umsetzungsprodukten mit monomerem Formaldehyd, Methylenglykol und/oder Polyoxymethylenglykolen mit Verbindungen (auch Formaldehyd selbst) die mit monomeren Formaldehyd, Methylenglykol und/oder Polyoxymethylenglykolen reaktiv sind, wobei eine erfingungsgemäße wässrige- Formaldehydlösung eingesetzt wird.

Bevorzugte Verbindungen, die mit monomerem Formaldehyd, Methylenglykol und/oder Polyoxymethylenglykolen reaktiv sind, sind ausgewählt aus der Gruppe bestehend aus:

- Aminogruppen enthaltenden Verbindungen, wobei Schiff'sche Basen entstehen bzw. eine Mannich-Reaktion durchgeführt wird. Beispielsweise reagieren Amine mit den erfindungsgemäßen wässrigen Formaldehydlösungen und Wasserstoff zu Methylaminen. Mit Ammoniak können Hexamethylentetramine hergestellt werden und mit Amoniumchlorid erfolgt die Bildung von Monomethylamin, Dimethylamin oder Trimethylamin und Ameisensäure, in Abhängigkeit von den Reaktionsbedingungen. Durch Umsetzung der erfindungsgemäßen wässrigen Formaldehydlösungen mit Ammoniak und Ketonen können Imidazole hergestellt werden. Durch Umsetzung mit Harnstoff werden Mono-, Di- und Trimethylolharnstoffe erhalten, mit Melamin erfolgt die Bildung von Methylolmelaminen, wobei durch Polykondensation von Melamin mit den erfindungsgemäßen wässrigen Formaldehydlösungen Melaminharze gebildet werden.

- Diolen, wobei durch die Umsetzung mit den erfindungsgemäßen wässrigen Formaldehydlösungen cyclische Ether gebildet werden, beispielsweise entsteht aus Glykol und den erfindungsgemäßen wässrigen Formaldehydlösungen Dioxolan; sowie Alkoholen, Thiolen, Carbonsäuren,

- Aldehyden (einschließlich Formaldehyd selbst), wobei durch Aldorisierungsreaktion mehrwertige Alkohole wie Zukker, Pentaerythrit, Trimethylolpropan und Neopentylglykol gebildet werden.

- Malonaten oder Ketonen (sowie primären Aldehyden), die eine $CH_2$-Gruppe in Nachbarschaft zur Carbonylgruppe tragen, wobei Doppelbindungen gebildet werden.

- Hydroxylaminen, Hydrazinen oder Semicarbaziden, wobei Formaldehydoxime, die entsprechenden Hydrazone oder Semicarbazone gebildet werden.

- Acetylen, wobei in einer Reppe-Addition 2-Butin-1,4-diol gewonnen wird, das zu Butandiol weiter hydriert werden kann.

- Aromatischen Verbindungen wie Benzol, Anilin oder Toluidin, wobei die entsprechenden Diphenylmethanderivate, z.B. Diaminodiphenylmethan (MDA), gebildet werden.

- Olefinen, wobei in einer säurekatalysierten Prins-Reaktion $\alpha$-Hydroxymethylverbindungen hergestellt werden, wobei die aromatischen Verbindungen und Olefine jeweils neben den genannten funktionellen Gruppen oder an deren Stelle andere oder keine funktionellen Gruppen tragen können.

Weitere wichtige Reaktionen sind die Trimerisierung von Formaldehyd, wobei Trioxan gebildet wird. Dabei wird die erfindungsgemäße wässrige Formaldehydlösung in einem Reaktor in Gegenwart eines sauren Katalysators umgesetzt. Eine Trioxan-Formaldehyd-Wasser-Mischung wird abgetrennt, aufkonzentriert und daraus Trioxan mit einem inerten Lösungsmittel extrahiert. Die Wasser-Formaldehyd-Fraktion wird zum Prozeßbeginn zurückgeführt. Dabei ist der Einsatz der erfindungsgemäßen wässrigen Formaldehydlösungen aufgrund der hohen Konzentration und des niedrigen Wassergehaltes besonders vorteilhaft, da zum Verdampfen von Wasser in diesem Verfahren viel Energie benötigt wird.

Die Polymerisation von Formaldehyd oder cyclischen Acetalen wie 1,3,5,-Trioxan führt zu Polyoxymethylenen (POM). Eine Copolymerisation von Trioxan mit cyclischen Ethern, z.B. Ethylenoxid führt zur Bildung von modifizierten POMs. Solche POMs können durch Gasphasen-, Fällungs-, Lösungs- oder Massen-Polymerisation aus den erfindungsgemäßen wässrigen Formaldehydlösungen hergestellt werden.

Durch Kondensation der erfindungsgemäßen wässrigen Formaldehydlösungen mit Harnstoff, Melamin (wie bereits erwähnt), Urethanen, Cyanamid, aromatischen Sulfonamiden, Aminen und Phenol können eine Vielzahl von Kunststoffen (Kunstharzen) hergestellt werden.

Des weiteren kann aus den erfindungsgemäßen wässrigen Formaldehydlösungen und CO Glykolsäure hergestellt werden. Durch Umsetzung von Hydrocyanolsäure mit den wässrigen Formaldehydlösungen gemäß der vorliegenden Erfindung können Glykolnitrile hergestellt werden.

Diese Listung ist nicht vollständig. Lehrbücher der organischen Chemie und der technischen Chemie (z.B. Ullmann's Encyclopedia of Industrial Chemistry, 6. Edition, 2000 electronic release, Stichwort: Formaldehyde; Kap. 3 (Chemical Properties) oder J. Frederic Walker "Formaldehyde" /American Chemical Society monographs series 1964 enthalten weitere Beispielreaktionen. Durch diese Listung soll aber beispielhaft die industrielle Bedeutung des Formaldehyds als Synthesebaustein im gesamten Bereich der organischen Chemie verdeutlicht werden. Dies betrifft sowohl kleintonnagie Zwischenprodukte im Pharma- oder Pflanzenschutzbereich, z.B. Oxime, als auch großtonnagie Produkte wie Diphenylmethanderivate.

Ein weiterer Gegenstand der vorliegenden Anmeldung ist daher die Verwendung der erfindungsgemäßen wässrigen Formaldehydlösungen zur Herstellung von Kunststoffen (z.B. Kunstharzen, Aminoplasten); Herstellung von Düngemitteln; zur Synthese zahlreicher organischer Verbindungen (z.B. Hexamethylentetramin, mehrwertige Alkohole wie Pentaerythrit, Trimethylolpropan, Neopentylglykol, Diphenylmethanderivate, Oxime, cyclische Ether, Trioxan und Butindiol, $\alpha$-Hydroxy-Methylverbindungen, Glykolnitrile); Herstellung von Farbstoffen (z.B. Fuchsin); in Holzharz-Leimen als Phenol-Formaldehydharz und zur Herstellung von Polyoxymethylen und modifizierten Polyoxymethylenen.

In allen genannten Herstellungsverfahren ist der Einsatz der erfindungsgemäßen wässrigen Formaldehydlösungen möglich. Dabei wird die Wasserlast im Vergleich zum Einsatz der bisher benutzten wässrigen Formaldehydlösungen deutlich vermindert, wodurch die Raum-Zeit-Ausbeute erhöht wird und die Investitionskosten durch die Verwendung kleiner Apparate gesenkt werden. Die Menge des anfallenden Abwassers wird reduziert und bei der thermischen Abtrennung von Wasser werden Energieeinsparungen erzielt.

Die nachfolgenden Beispiele erläutern die Erfindung zusätzlich.

**Beispiele**

Herstellung von hochkonzentrierten Formaldehydlösungen in einem Dünnschichtverdampfer:

Über eine Zuleitung wurde eine 30 gew.-%-ige Formaldehyd-Rohlösung bei einer Temperatur von 20 °C zugeführt.

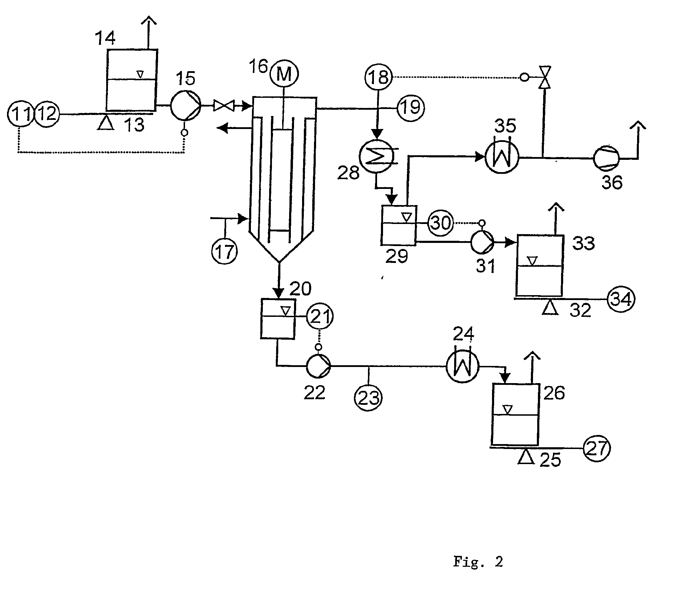

Figur 2 zeigt einen Laboraufbau eines Filmverdampfers zur Herstellung hochkonzentrierter Formaldehydlösungen.

Darin bedeuten:

| | |
|---|---|
| 11 | Flußregelung und -anzeige |
| 12 | Wägung mit Anzeige und Aufzeichnung |
| 13 | Waage 1 |
| 14 | Behälter 1 |
| 15 | Pumpe 1 |
| 16 | Filmverdampfer |
| 17 | Temperaturmessstelle mit Anzeige, Aufzeichnung und Regelung |
| 18 | Druckmessung mit Anzeige, Aufzeichnung und Regelung |
| 19 | Temperaturmessstelle mit Anzeige und Aufzeichnung |
| 20 | Behälter 2 |
| 21 | Füllstandsmessung mit Anzeige und Regelung |
| 22 | Pumpe 2 |
| 23 | Analyse mit Anzeige und Aufzeichnung |
| 24 | Wärmeaustauscher 1 |
| 25 | Waage 2 |
| 26 | Behälter 3 |
| 27 | Wägung mit Anzeige und Aufzeichnung |
| 28 | Wärmeaustascher 2 |
| 29 | Behälter 4 |
| 30 | Füllstandsmessung mit Anzeige und Regelung |
| 31 | Pumpe 3 |
| 32 | Waage 3 |

33 Behälter 5
34 Wägung mit Anzeige und Aufzeichnung
35 Wärmeaustascher 3
36 Vakuumpumpe 1

**Beispiel 1**

Herstellung einer 77 gew.-%-igen Formaldehydlösung

| Laufende Nummer: | | 1 | 2 | 3 |
|---|---|---|---|---|
| **Bezeichnung** | | Zulauf | Sumpf | Kondensat |
| **Gesamtmenge** | g/h | 789,6 | 252,6 | 537,0 |
| **Menge Wasser** | g/h | 552,7 | 58,1 | 494,6 |
| **Menge CH$_2$O** | g/h | 236,9 | 194,5 | 42,4 |
| **Konzentration CH$_2$O** | g/g | 0,30 | 0,77 | 0,079 |
| | | | | |
| **Parameter**: | | | | |
| **Druck** | mbar | 70 | | |
| **Drehzahl** | 1/min | 400 | | |
| **Temperatur** | °C | 47,9 | | |
| **Temperatur Heizmantel** | °C | 90,0 | | |
| **Temperatur Kondensator** | °C | 2,0 | | |

**Beispiel 2**

Herstellung einer 69 Gew.-%-igen Formaldehydlösung

| Laufende Nummer: | | 1 | 2 | 3 |
|---|---|---|---|---|
| **Bezeichnung** | | Zulauf | Sumpf | Kondensat |
| **Gesamtmenge** | g/h | 1027,2 | 245,3 | 781,9 |
| **Menge Wasser** | g/h | 816,6 | 76,0 | 740,5 |
| **Menge CH$_2$O** | g/h | 210,6 | 169,3 | 41,4 |
| **Konzentration CH$_2$O** | g/g | 0,205 | 0,690 | 0,053 |
| | | | | |
| **Parameter**: | | | | |
| **Druck** | mbar | 80 | | |
| **Drehzahl** | 1/min | 400 | | |
| **Temperatur** | °C | 64,5 | | |
| **Temperatur Heizmantel** | °C | 95 | | |
| **Temperatur Kondensator** | °C | 3 | | |

**Patentansprüche**

1.  Wässrige Formaldehydlösung enthaltend Formaldehyd in Form von monomerem Formaldehyd, Methylenglykol und Polyoxymethylenglykolen in einer Gesamtkonzentration x von $\geq$ 65 Gew.-%, **dadurch gekennzeichnet, dass** die mittlere Molmasse $\overline{M}$ der Polyoxymethylenglykole in Abhängigkeit von der Formaldehydkonzentration gleich oder kleiner den gemäß Formel I erhaltenen Werten ist:

$$\left(\frac{\overline{M}}{g/mol}\right) = 48 + 6,589 \cdot 10^{-1} \cdot \left(\frac{x}{Gew.-\%}\right) + 4,725 \cdot 10^{-2} \cdot \left(\frac{x}{Gew.-\%}\right)^2 - 3,434 \cdot 10^{-3} \cdot \left(\frac{x}{Gew.-\%}\right)^3$$

$$+ 9,625 \cdot 10^{-5} \cdot \left(\frac{x}{Gew.-\%}\right)^4 - 1,172 \cdot 10^{-6} \cdot \left(\frac{x}{Gew.-\%}\right)^5 + 5,357 \cdot 10^{-9} \cdot \left(\frac{x}{Gew.-\%}\right)^6 \cdot$$

$$(I)$$

darin bedeuten:

$\overline{M}$ mittlere Molmasse
x Gesamtkonzentration an Formaldehyd in Form von monomerem Formaldehyd, Methylenglycol und Polyoxymethylenglycolen in Gew.-% (Formaldehydgesamtkonzentration).

2.  Wässrige Formaldehydlösung nach Anspruch 1, **dadurch gekennzeichnet, dass** kein Feststoffausfall in einem Zeitraum von mindestens 5 Sekunden, bevorzugt mindestens 1 Minute, besonders bevorzugt mindestens 5 Minuten, ganz besonders bevorzugt mindestens 1 Stunde auftritt.

3.  Wässrige Formaldehydlösung nach einem der Ansprüche 1 bis 2, **dadurch gekennzeichnet, dass** die Lösung Stabilisatoren ausgewählt aus Methanol, Ethanol, Propanol, Butanol, Harnstoff und Melamin enthält.

4.  Verfahren zur Herstellung einer wässrigen Formaldehydlösung gemäß einem der Ansprüche 1 bis 3 durch einstufige thermische Auftrennung einer wässrigen Formaldehydlösung enthaltend 5 bis 65 Gew.-% eines Ausgangsgemisches an Formaldehyd in Form von monomerem Formaldehyd, Methylenglykol und Polyoxymethylenglykolen sowie gegebenenfalls weitere Komponenten wie Stabilisatoren, wobei die wässrige Formaldehydlösung zumindest teilweise verdampft wird, in wenigstens zwei Fraktionen, in denen unterschiedliche Verbindungen des Gemisches im Vergleich zu dem Ausgangsgemisch im Gemisch angereichert sind, wobei in wenigstens einer der wenigstens zwei Fraktionen Wasser im Vergleich zum Ausgangsgemisch abgereichert ist, so dass der Formaldehyd in Form von monomerem Formaldehyd, Methylenglykol und Polyoxymethylenglykolen in dieser Fraktion in einer Gesamtkonzentration $\geq$ 65 Gew.-% vorliegt, wobei im Anschluss an die Auftrennung keine Alterung der Fraktion, worin Formaldehyd in Form von monomerem Formaldehyd, Methylenglykol und Polyoxymethylenglykolen in einer Gesamtkonzentration von $\geq$65 Gew.-% vorliegt, bei erhöhten Temperaturen erfolgt.

5.  Verfahren nach Anspruch 4, **dadurch gekennzeichnet, dass** die Auftrennung in einem Zeitraum von 1 Sekunde bis 5 Stunden erfolgt.

6.  Verfahren nach Anspruch 4 oder 5, **dadurch gekennzeichnet, dass** die zumindest teilweise Verdampfung in einem Filmverdampfer, bevorzugt einem Filmverdampfer ausgewählt aus Dünnschichtverdampfer, Wendelrohr- bzw. Schlängenrehrverdämpfer und Fallfilmverdampfer, erfolgt.

7.  Verfahren nach Anspruch 4 oder 5, **dadurch gekennzeichnet, dass** die zumindest teilweise Verdampfung in einer Reaktionskolonne erfolgt.

8.  Verfahren nach Anspruch 4, **dadurch gekennzeichnet, dass** die thermische Auftrennung bei Temperaturen von 10 bis 150°C geschieht

9.  Verfahren zur Herstellung von monomeren, oligomeren oder polymeren Umsetzungsprodukten mit monomerem Formaldehyd, Methylenglykol und/oder Polyoxymethylenglykolen mit Verbindungen (auch Formaldehyd selbst), die mit monomerem Formaldehyd, Methylenglykol und/oder Polyoxymethylenglykolen reaktiv sind, **dadurch gekennzeichnet, dass** eine wässrige Formaldehydlösung gemäß einem der Ansprüche 1 bis 3 eingesetzt wird.

10. Verfahren nach Anspruch 9, **dadurch gekennzeichnet, dass** die Verbindungen, die mit monomerem Formaldehyd, Methylenglykol und/oder Polyoxymethylenglykolen reaktiv sind, ausgewählt sind aus der Gruppe bestehend aus Aminogruppen enthaltenden Verbindungen; Alkoholen, Thiolen, Carbonsäuren, Diolen, Aldehyden (einschließlich Formaldehyd selbst); Malonaten; Ketonen; Hydroxylaminen, Hydrazinen, Semicarbaziden; Acetylen und aromatischen Verbindungen und Olefinen, die jeweils neben den genannten funktionellen Gruppen oder an deren Stelle andere oder keine funktionellen Gruppen tragen können.

11. Verwendung von wässrigen Formaldehydlösungen gemäß einem der Ansprüche 1 bis 3 zur Herstellung von Kunststoffen; zur Herstellung von Düngemitteln; zur Synthese zahlreicher organischer Verbindungen wie Diaminodiphenylmethan (MDA); zur Herstellung von Farbstoffen; in Holzharz-Leimen als Phenol-Formaldehydharz und zur Herstellung von Polyoxymethylen und modifizierten Polyoxymethylenen.

**Claims**

1. An aqueous formaldehyde solution comprising formaldehyde in the form of monomeric formaldehyde, methylene glycol and polyoxymethylene glycols in a total concentration x of $\geq$ 65% by weight, wherein the mean molar mass $\overline{M}$ of the polyoxymethylene glycols is, as a function of the formaldehyde concentration, equal to or less than the values given by equation I:

$$\left(\frac{\overline{M}}{g/mol}\right) = 48 + 6.589 \cdot 10^{-1} \cdot \left(\frac{x}{\% \text{ by weight}}\right) + 4.725 \cdot 10^{-2} \cdot \left(\frac{x}{\% \text{ by weight}}\right)^2 - 3.434 \cdot 10^{-3} \cdot \left(\frac{x}{\% \text{ by weight}}\right)^3$$
$$+ 9.625 \cdot 10^{-5} \cdot \left(\frac{x}{\% \text{ by weight}}\right)^4 - 1.172 \cdot 10^{-6} \cdot \left(\frac{x}{\% \text{ by weight}}\right)^5 + 5.357 \cdot 10^{-9} \cdot \left(\frac{x}{\% \text{ by weight}}\right)^6$$

$$(I)$$

where:

$\overline{M}$ is the mean molar mass, and
x is the total concentration of formaldehyde in the form of monomeric formaldehyde, methylene glycol and polyoxymethylene glycols in % by weight (total formaldehyde concentration).

2. The aqueous formaldehyde solution according to claim 1, wherein no precipitation of solids occurs within a period of at least 5 seconds, preferably at least 1 minute, particularly preferably at least 5 minutes, very particularly preferably at least 1 hour.

3. The aqueous formaldehyde solution according to claim 1 or 2 which further comprises stabilizers selected from among methanol, ethanol, propanol, butanol, urea and melamine.

4. A process for preparing an aqueous formaldehyde solution according to any of claims 1 to 3 by single-stage thermal separation of an aqueous formaldehyde solution comprising from 5 to 65% by weight of a starting mixture of formaldehyde in the form of monomeric formaldehyde, methylene glycol and polyoxymethylene glycols and optionally further components such as stabilizers, in which the aqueous formaldehyde solution is at least partly vaporized, into at least two fractions in which various compounds of the mixture are present in higher concentrations than in the starting mixture, where at least one of the two or more fractions is depleted in water compared to the starting mixture so that the formaldehyde in the form of monomeric formaldehyde, methylene glycol and polyoxymethylene glycols is present in this fraction in a total concentration of $\geq$ 65% by weight and no aging of the fraction in which formaldehyde in the form on monomeric formaldehyde, methylene glycol and polyoxymethylene glycols is present in a total concentration of $\geq$ 65% by weight at elevated temperatures occurs after the separation.

5. The process according to claim 4, wherein the separation is carried out within a period of from 1 second to 5 hours.

6. The process according to claim 4 or 5, wherein the at least partial vaporization is carried out in a film evaporator, preferably a film evaporator selected from among thin film evaporators, helical tube or coiled tube evaporators and falling film evaporators.

7. The process according to claim 4 or 5, wherein the at least partial vaporization is carried out in a reaction column.

8. The process according to claim 4, wherein the thermal separation is carried out at from 10 to 150°C.

9. A process for preparing monomeric, oligomeric and polymeric reaction products of monomeric formaldehyde, methylene glycol and/or polyoxymethylene glycols with compounds (including formaldehyde itself) which react with monomeric formaldehyde, methylene glycol and/or polyoxymethylene glycols, wherein an aqueous formaldehyde solution according to any of claims 1 to 3 is used.

10. The process according to claim 9, wherein the compounds which react with monomeric formaldehyde, methylene glycol and/or polyoxymethylene glycols are selected from the group consisting of compounds comprising amino groups; alcohols, thiols, carboxylic acids, diols, aldehydes (including formaldehyde itself); malonates; ketones; hydroxylamines, hydrazines, semicarbazides; acetylene and aromatic compounds and olefins which may in each case bear other or no functional groups in addition to or in place of the functional groups mentioned.

11. The use of an aqueous formaldehyde solution according to any of claims 1 to 3 for preparing synthetic polymers; for preparing fertilizers; for the synthesis of numerous organic compounds such as diaminodiphenylmethane (MDA); for preparing dyes; in wood glues as phenol-formaldehyde resin and for preparing polyoxymethylene and modified polyoxymethylenes.

**Revendications**

1. Solution aqueuse de formaldéhyde, contenant du formaldéhyde sous forme de formaldéhyde monomère, de méthylèneglycol et de polyoxyméthylèneglycols en concentration totale x ≥ 65 % en poids, **caractérisée en ce que** la masse molaire moyenne $\overline{M}$ des polyoxyméthylèneglycols est, en fonction de la concentration de formaldéhyde, inférieure ou égale aux valeurs obtenues selon la formule I :

$$\left(\frac{\overline{M}}{\text{g/mole}}\right) = 48 + 6,589 \cdot 10^{-1} \cdot \left(\frac{x}{\text{% en poids}}\right) + 4,725 \cdot 10^{-3} \cdot \left(\frac{x}{\text{% en poids}}\right)^3 - 3,434 \cdot 10^{-5} \cdot \left(\frac{x}{\text{% en poids}}\right)^4$$

$$+ 9,625 \cdot 10^{-5} \cdot \left(\frac{x}{\text{% en poids}}\right)^5 - 1,172 \cdot 10^{-6} \cdot \left(\frac{x}{\text{% en poids}}\right)^5 + 5,357 \cdot 10^{-9} \cdot \left(\frac{x}{\text{% en poids}}\right)^6 .$$

$$\text{(I)}$$

dans laquelle :

$\overline{M}$ représente la masse molaire moyenne,
x représente la concentration totale en formaldéhyde sous forme de formaldéhyde monomère, de méthylèneglycol et de polyoxyméthylèneglycols, exprimée en % en poids (concentration totale de formaldéhyde).

2. Solution aqueuse de formaldéhyde selon la revendication 1, **caractérisée en ce qu'**il ne se produit aucune précipitation de solides sur un intervalle de temps d'au moins 5 secondes, de préférence, d'au moins 1 minute, mieux encore, d'au moins 5 minutes, bien mieux encore, d'au moins 1 heure.

3. Solution aqueuse de formaldéhyde selon l'une quelconque des revendications 1 à 2, **caractérisée en ce que** la solution contient des stabilisateurs choisis parmi le méthanol, l'éthanol, le propanol, le butanol, l'urée et la mélamine.

4. Procédé de fabrication d'une solution aqueuse de formaldéhyde selon l'une quelconque des revendications 1 à 3, consistant à effectuer en une étape la séparation thermique d'une solution aqueuse de formaldéhyde contenant 5 à 65 % en poids d'un mélange de départ de formaldéhyde sous forme de formaldéhyde monomère, de méthylèneglycol et de polyoxyméthylèneglycols, ainsi que, éventuellement, d'autres composants, tels que des stabilisateurs, la solution aqueuse de formaldéhyde étant soumise à une évaporation au moins partielle de manière à générer au moins deux fractions, dans lesquelles divers composés du mélange sont enrichis dans le mélange en comparaison du mélange de départ, l'eau étant appauvrie dans au moins l'une des au moins deux fractions en comparaison du mélange de départ, de sorte que le formaldéhyde sous forme de formaldéhyde monomère, de méthylèneglycol et de polyoxyméthylèneglycols se présente, dans cette fraction, en concentration totale ≥ 65 % en poids, aucun vieillissement de la fraction dans laquelle se trouve le formaldéhyde sous forme de formaldéhyde monomère, de méthylèneglycol et de polyoxyméthylèneglycols en concentration totale ≥ 65 % en poids ne se produisant à des températures plus élevées, après l'étape de séparation.

5. Procédé selon la revendication 4, **caractérisé en ce que** la séparation est effectuée dans un intervalle de temps s'étendant de 1 seconde à 5 heures.

6. Procédé selon la revendication 4 ou 5, **caractérisé en ce que** l'on réalise l'évaporation au moins partielle dans un évaporateur à film, de préférence, dans un évaporateur à film choisi parmi un évaporateur à couche mince, un évaporateur à tube spiralé ou à tube hélicoïdal et un évaporateur à film tombant.

7. Procédé selon la revendication 4 ou 5, **caractérisé en ce que** l'on réalise l'évaporation au moins partielle dans une colonne réactionnelle.

8. Procédé selon la revendication 4, **caractérisé en ce que** la séparation thermique est réalisée à des températures de 10 à 150 °C.

9. Procédé de fabrication de produits réactionnels monomères, oligomères ou polymères avec du formaldéhyde monomère, du méthylèneglycol et/ou des polyoxyméthylèneglycols avec des composés (dont le formaldéhyde lui-même) qui sont réactifs avec le formaldéhyde monomère, le méthylèneglycol et/ou les polyoxyméthylène-glycols, **caractérisé en ce que** l'on utilise une solution aqueuse de formaldéhyde selon l'une quelconque des revendications 1 à 3.

10. Procédé selon la revendication 9, **caractérisé en ce que** les composés, qui sont réactifs avec le formaldéhyde monomère, le méthylèneglycol et/ou les polyoxyméthylèneglycols, sont choisis dans le groupe constitué des composés contenant des groupements amino ; des alcools, des thiols, des acides carboxyliques, des diols, des aldéhydes (y compris le formaldéhyde lui-même) ; des malonates ; des cétones ; des hydroxylamines, des hydrazines, des semi-carbazides ; de l'acétylène, des composés aromatiques et des oléfines, qui peuvent porter respectivement, outre les groupements fonctionnels cités, ou à la place de ceux-ci, d'autres groupements fonctionnels, ou qui peuvent ne porter aucun groupement fonctionnel.

11. Utilisation de solutions aqueuses de formaldéhyde selon l'une quelconque des revendications 1 à 3, pour la fabrication de matériaux synthétiques ; pour la fabrication d'engrais ; pour la synthèse de bon nombre de composés organiques, tels que le diaminodiphénylméthane (MDA) ; pour la fabrication de colorants ; dans des colles de colophane comme résine de phénol-formaldéhyde et pour la fabrication de polyoxyméthylène et de polyoxyméthy-lènes modifiés.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 1190682 A

- EP 1063221 A

**In der Beschreibung aufgeführte Nicht-Patentliteratur**

- Ullmann's Encyclopedia of Industrial Chemistry. 2000, 2
- **HAHNENSTEIN, I. ; ALBERT, M. ; HASSE, H. ; KREITER, C.G. ; MAURER, G.** NMR Spectroscopic and Densimetric Study of Reaction Kinetics of Formaldehyde Polymer Formation in Water, Deuterium Oxide, and Methanol. *Ind. Eng. Chem. Res.,* 1995, vol. 34, 440-450
- **J. F. WALKER.** Formaldehyde. Reinhold Publ. Comp, 1953, 382

- *Chem. Ing. Tech.,* 1996, vol. 68 (6), 706-710
- *Chem. Ing. Tech.,* 1970, vol. 42 (6), 349-354
- Formaldehyde. Ullmann's Encyclopedia of Industrial Chemistry. 2000, vol. 6
- **J. FREDERIC WALKER.** Formaldehyde. American Chemical Society, 1964